# EUROPEAN PATENT APPLICATION

(11) **EP 3 636 772 A1**
(43) Date of publication of application: **15.04.2020**
(21) Application number: 18812953.0
(22) Date of filing: 25.05.2018
(51) Int. Cl.: C12Q 1/68, C12N 15/113

(54) **TWO MOLECULAR MARKERS FOR DIAGNOSIS OF GLAUCOMA, KIT, AND APPLICATION**

(30) Priority: 07.06.2017 CN 201710423729; 07.06.2017 CN 201710423183
(71) Applicant: The Second Xiangya Hospital Of Central South University, Hunan 410011 (CN)
(72) Inventor: JIANG, Bing, Changsha Hunan 410011 (CN); XIE, Lili, Changsha Hunan 410011 (CN); ZHANG, Lusi, Changsha Hunan 410011 (CN); HUANG, Wei, Changsha Hunan 410011 (CN)
(74) Representative: Petraz, Gilberto Luigi
(86) International application number: PCT/CN2018/088322
(87) International publication number: WO 2018/223846

(57) **Abstract**

The present invention discloses two molecular markers, kits and applications for glaucoma diagnosis. Compared with people in a control group, the expressions of lncRNAs: ENST00000607393 are all up-regulated in aqueous humors, irises and serums of patients with glaucoma, and the expression of lncRNAs: ENST00000508241 is up-regulated in aqueous humors of patients with glaucoma. It indicates that ENST00000607393 and ENST00000508241 are highly expressed lncRNAs in glaucoma and are biomarkers that contribute to the diagnosis of glaucoma. The present invention provides a strong molecular biology basis for the diagnosis of glaucoma, and has far-reaching clinical significance and generalization performance.

Free contents of sequence tables
<110> The Second Xiangya Hospital of Central South University

<120> Two molecular markers, kits and applications for
glaucoma diagnosis

<130> Null

<160> 6

<170> PatentIn version 3.3

<210> 1

<211> 312

<212> DNA

<213> Sequence of lncRNA: ENST00000607393

<400> 1

ctagcttccg agggacttct ctgcgagcac cgcaggcgtg tgcatttctt cgaatctgag 60

aattccagcg aactcgggct ctaccagagt ttaagtcggc ccctggggcg tctgtttgtt 120

tattggctct taagggaaaa aagtttgaag atctttaacc agcctcagac ctggcgcggg 180

cggaaatccc ggaatctccc caagcaaaag tgatataact tcaagtcgtt atataagtgg 240

ggggctcctt cttcccctcc ctacgccgag ccttctcatt atcattccgg gagtaaaatg 300

tgtctcgctc aa 312

<210> 2

<211> 21

<212> DNA

<213> Sequence F of primer specifically amplifying lncRNAs
ENST00000607393

<400> 2

ggcgtctgtt tgtttattgg c 21

<210> 3

<211> 21

<212> DNA

<213> Sequence R of primer specifically amplifying lncRNAs
ENST00000607393

<400> 3

tcacttttgc ttggggagat t 21

<210> 4

<211> 540

<212> DNA

<213> Sequence of lncRNA: ENST00000508241

<400> 4

aaggtacact ctactatgta tgcacagcca cagaaaaatc atagtgtgta cattgcttga 60

atgatccttt tgggtttggc tgttactcac agatgaagga gacatttcca aggttgtaac 120

cagagtcatt ctaagcacag tccttgcagc agtagagaca atcccaatga ctacaggaaa 180

tcctcaactc tcatccagaa cctgtaagct caggacatgc atcataggaa ggatgtgaat 240

tgctgaagtg cttccatggt agtcttttgc tccctgccta cgtgggcaag aagctgtaga 300

ctctgggata atttggacga tcccagcacg ggtatactcc actctgcttc atggcactta 360

ccaaggctgg agagagacca cacccatccc ttctgttttc cattgcattc ccttccccaa 420

actttccatc cagaaattct tctttggttg accctgccac attgtatatt cttgttctcc 480

ccccatgatg cattttattt cagtatcctt agattcctaa taaatcaatt atttaaacat 540

<210> 5

<211> 21

<212> DNA

<213> Sequence F of primer specifically amplifying lncRNAs
ENST00000508241

<400> 5

tttgggtttg gctgttactc a                                21

<210> 6

<211> 22

<212> DNA

<213> Sequence R of primer specifically amplifying lncRNAs
      ENST00000508241

<400> 6

gaccacattt cccaagaaca ct                               22

SEQUENCE LISTING

<110>  The Second Xiangya Hospital of Central South University

<120>  Two molecular markers,kits and applications for glaucoma diagnosis

<130>  Null

<160>  6

<170>  PatentIn version 3.3


<210>  1
<211>  312
<212>  DNA
<213>  Sequence of lncRNA: ENST00000607393

<400>  1
ctagcttccg agggacttct ctgcgagcac cgcaggcgtg tgcatttctt cgaatctgag     60

aattccagcg aactcgggct ctaccagagt ttaagtcggc ccctggggcg tctgtttgtt    120

tattggctct taagggaaaa aagtttgaag atctttaacc agcctcagac ctggcgcggg    180

cggaaatccc ggaatctccc caagcaaaag tgatataact tcaagtcgtt atataagtgg    240

ggggctcctt cttcccctcc ctacgccgag ccttctcatt atcattccgg gagtaaaatg    300

tgtctcgctc aa                                                        312


<210>  2
<211>  21
<212>  DNA
<213>  Sequence F of primer specifically amplifying lncRNAs ENST00000607393

<400>  2
ggcgtctgtt tgtttattgg c                                               21


<210>  3
<211>  21
<212>  DNA
<213>  Sequence R of primer specifically amplifying lncRNAs ENST00000607393

<400>  3
tcacttttgc ttggggagat t                                               21

<210>  4
<211>  540
<212>  DNA
<213>  Sequence of lncRNA: ENST00000508241

<400>  4
aaggtacact ctactatgta tgcacagcca cagaaaaatc atagtgtgta cattgcttga     60

atgatccttt tgggtttggc tgttactcac agatgaagga gacatttcca aggttgtaac    120

cagagtcatt ctaagcacag tccttgcagc agtagagaca atcccaatga ctacaggaaa    180

tcctcaactc tcatccagaa cctgtaagct caggacatgc atcataggaa ggatgtgaat    240

tgctgaagtg cttccatggt agtcttttgc tccctgccta cgtgggcaag aagctgtaga 300

ctctgggata atttggacga tcccagcacg ggtatactcc actctgcttc atggcactta 360

ccaaggctgg agagagacca cacccatccc ttctgttttc cattgcattc ccttccccaa 420

actttccatc cagaaattct tctttggttg accctgccac attgtatatt cttgttctcc 480

ccccatgatg cattttattt cagtatcctt agattcctaa taaatcaatt atttaaacat 540

<210> 5
<211> 21
<212> DNA
<213> Sequence F of primer specifically amplifying lncRNAs ENST00000508241

<400> 5
tttgggtttg gctgttactc a 21

<210> 6
<211> 22
<212> DNA
<213> Sequence R of primer specifically amplifying lncRNAs ENST00000508241

<400> 6
gaccacattt cccaagaaca ct 22

## Description

### Technical Field

The present invention relates to the technical field of molecular diagnosis, and in particular to long non-coding RNA markers, kits and applications that contribute to the diagnosis of glaucoma.

### Related Art

Eyes are very important sensory organs of a human body which can receive external light stimulation and transmit light impulse to the brain center to cause vision. Da Vinci once said: "the eyes are the windows of the soul, people can embrace and appreciate the infinite beauty of the world through the eyes, and the soul can live in the body peacefully." In the information age, about 90% of the information that people get from the outside world through sensory organs is done by the eyes. According to the data of the World Health Organization, ophthalmic diseases have become the third diseases that endanger and affect people's quality of life after cancer and cardiovascular diseases. Among all ophthalmic diseases, glaucoma is the first irreversible blindness-causing eye disease, it affects the visual quality by causing blindness through threatening and damaging the optic nerve and its pathways, thus seriously threatening human visual health, and causing incalculable losses to individuals, families and society.

The main hazard caused by glaucoma is to affect the visual function. Even in developed countries, only about 50% of patients with glaucoma can be timely diagnosed and treated, and the pathogenic factors and genetics laws of glaucoma are unknown. Therefore, it is necessary to scientifically grasp the law of its occurrence and development, so as to carry out early diagnosis and early treatment to avoid blindness in patients with glaucoma. At present, the diagnosis of glaucoma mainly depends on medical history, morphological and functional examinations, such as intraocular tension measurement, ultrasound biomicroscopy, fundus photography, optical coherence tomography and visual field examination. Although these examinations can diagnose glaucoma, the research shows that the patient with glaucoma diagnosed by morphological and functional means has the visual function impairment of more than 50%. The biochemical examination, serological screening and detection criteria related to glaucoma are still in a relatively blank state. Therefore, it is especially important to find a marker with high sensitivity and high specificity for the diagnosis and monitoring of glaucoma.

The genome project research shows that in the 3 billion base pairs that make up the human genome, only 1.5% of nucleotide sequences are used for protein coding, and the remaining 98.5% of genomes are non-protein coding sequences. These sequences were once considered to be "garbage sequences" accumulated during evolution and were not taken into account. In the encyclopedia of DNA elements (ENCODE) project of human genome, which was subsequently launched, the research shows that 75% of gene sequences can be transcribed into RNA, and nearly 74% of transcription products are non-coding RNAs (ncRNAs). In ncRNAs, transcripts with a sequence length greater than 200 bases are long non-coding RNAs (lncRNAs), which are attracting attention due to their functional richness and diversity of mechanisms of action. lncRNAs can regulate the expression of protein coding genes at various levels of transcription, post-transcription and translation, and thus participate extensively in important life processes including cell differentiation and body development, and the abnormal expression thereof is also closely related to the occurrence of many major human diseases.

lncRNA is highly conserved and tissue-specific, and is abundant in the brain. lncRNA not only participates in the growth and development and function perfection of the nervous system to make the nervous system grow and develop according to a certain time sequence in a certain space, but also participates in executing the function of the nervous system. lncRNA participates in the development and function execution of the nervous system through various mechanisms, including gene imprinting, chromatin remodeling, cell cycle regulation, splicing regulation, mRNA degradation and translational regulation and other processes as a cis-acting element and a trans-acting factor. Therefore, it is a feasible means to reflect the physiological and pathological states of the nervous system by detecting changes in the composition and expression level of lncRNAs.

Aqueous humor belongs to the contents of the eyes and is produced by the ciliary body. It enters the blood through the pupil, trabecular meshwork, etc., and is in a dynamic circulation. Its composition is closely related to the local physiological and pathological environments of the eyeball. The exosome as a crucial mediator of intercellular communication in the body includes lncRNAs, mRNAs, proteins, and lipids, etc. The substances contained in the exosome can remain stable for a long time due to a special protection mechanism. Therefore, molecules such as lncRNAs in the aqueous humor can transmit intercellular communication information in the form of exosome, and can enter the blood along with the dynamic circulation of aqueous humor. Therefore, the glaucoma-related lncRNAs research has the potential to provide a theoretical basis for the detection of glaucoma-related biomarkers.

### SUMMARY

### Technical problems

The present invention aims to provide two molecular markers of lncRNAs ENST00000607393 and lncRNAs ENST00000508241, kits and applications for glaucoma diagnosis.

### Problem solution

### Technical solution

### Summary of the invention

A first objective of the present invention is to provide a long non-coding RNA for early diagnosis of glaucoma, which has great significance for the detection of glaucoma.

A molecular marker lncRNAs ENST00000607393 for early diagnosis of glaucoma has a sequence shown in SEQ ID NO: 1.

A second objective of the present invention is to provide application of the molecular marker lncRNAs ENST00000607393, and application of a product for detecting the expression level of lncRNAs ENST00000607393 in preparation of a tool for early diagnosis of glaucoma.

The product for detecting the expression level of lncRNAs ENST00000607393 includes a preparation for detecting the expression level of lncRNAs ENST00000607393 by real-time fluorescent quantitative PCR.

The preparation for detecting the expression level of lncRNAs ENST00000607393 by real-time fluorescent quantitative PCR includes primers specifically amplifying lncRNAs ENST00000607393.

The sequences of the primers specifically amplifying lncRNAs ENST00000607393 for detecting the expression level of lncRNAs ENST00000607393 by real-time fluorescent quantitative PCR are as follows:
F:5'-GGCGTCTGTTTGTTTATTGGC-3',
R:5'-TCACTTTTGCTTGGGGAGATT-3'.

A third objective of the present invention is to provide a kit for early diagnosis of glaucoma, including a reagent for detecting the expression level of lncRNAs ENST00000607393, and in particular including a reagent for detecting the expression level of IncRNAs ENST00000607393 by real-time fluorescent quantitative PCR.

The reagent for detecting the expression level of lncRNAs ENST00000607393 by real-time fluorescent quantitative PCR includes primers specifically amplifying lncRNAs ENST00000607393 by real-time fluorescent quantitative PCR.

The reagent for detecting the expression level of lncRNAs ENST00000607393 by real-time fluorescent quantitative PCR includes a pair of primers specifically amplifying lncRNAs ENST00000607393 by real-time fluorescent quantitative PCR, of which the primer sequences are as follows:
F:5'-GGCGTCTGTTTGTTTATTGGC-3',
R:5'-TCACTTTTGCTTGGGGAGATT-3'.

A qRT-PCR detection method is used to verify the expression levels of lncRNAs-T267384,ENST00000607393 and T342877 in aqueous humors, iris tissues and serums and analyze the difference of expression quantities thereof in the two types of aqueous humors. In the three tissues, the aqueous humor has the highest diagnostic significance, and the sensitivity/specificity of three lncRNAs for the diagnosis of glaucoma in the aqueous humor is all greater than 90%. The possible cause of the phenomenon is that the presence of a blood-aqueous humor barrier significantly limits the entry of blood-derived immune cells and signaling molecules from other parts of the whole body into the aqueous humor. Therefore, compared with serum, the aqueous humor can more specifically represent the physiological and pathological states of the eye. Therefore, in future research, the aqueous humor may have a greater diagnostic value for glaucoma than serum.

A fourth objective of the present invention is to provide another long non-coding RNA for early diagnosis of glaucoma, which has great significance for the detection of glaucoma.

A molecular marker lncRNAs ENST00000508241 for early diagnosis of glaucoma has a sequence shown in SEQ ID NO: 4.

A fifth objective of the present invention is to provide application of the molecular marker lncRNAs ENST00000508241, and application of a product for detecting the expression level of lncRNAs ENST00000508241 in preparation of a tool for early diagnosis of glaucoma.

The product for detecting the expression level of lncRNAs ENST00000508241 includes a preparation for detecting the expression level of lncRNAs ENST00000508241 by real-time fluorescent quantitative PCR.

The preparation for detecting the expression level of lncRNAs ENST00000508241 by real-time fluorescent quantitative PCR includes primers specifically amplifying lncRNAs ENST00000508241.

The sequences of the primers specifically amplifying lncRNAs ENST00000508241 for detecting the expression level of lncRNAs ENST00000508241 by real-time fluorescent quantitative PCR are as follows:
F:5'TTTGGGTTTGGCTGTTACTCA3'
R:5'GACCACATTTCCCAAGAACACT3'.

A sixth objective of the present invention is to provide another kit for early diagnosis of glaucoma, including a reagent for detecting the expression level of lncRNAs ENST00000508241, and in particular including a reagent for detecting the expression level of lncRNAs ENST00000508241 by real-time fluorescent quantitative PCR.

The reagent for detecting the expression level of lncRNAs ENST00000508241 by real-time fluorescent quantitative PCR includes primers specifically amplifying lncRNAs ENST00000508241 by real-time fluorescent quantitative PCR.

The reagent for detecting the expression level of lncRNAs ENST00000508241 by real-time fluorescent quantitative PCR includes a pair of primers specifically amplifying lncRNAs ENST00000508241 by real-time fluorescent quantitative PCR, of which the primer sequences are as follows:
F:5'TTTGGGTTTGGCTGTTACTCA3'
R:5'GACCACATTTCCCAAGAACACT3'.

### Beneficial effects of the invention

### Beneficial effects

For the present application, it is found by the applicants that the expressions of lncRNAs: ENST00000607393 are all up-regulated in the aqueous humors, irises and serums of patients with glaucoma compared with people in a control group. It indicates that ENST00000607393 is highly expressed lncRNAs in glaucoma and is a biomarker that contributes to the diagnosis of glaucoma. The present invention provides a strong molecular biology basis for the diagnosis of glaucoma, and has far-reaching clinical significance and generalization performance.

It is found by the applicants that the expression of lncRNAs: ENST00000508241 is up-regulated in the aqueous humors of patients with glaucoma compared with people in the control group. It indicates that ENST00000508241 is highly expressed lncRNAs in glaucoma and is a biomarker that contributes to the diagnosis of glaucoma. The present invention provides a strong molecular biology basis for the diagnosis of glaucoma, and has far-reaching clinical significance and generalization performance.

### BRIEF DESCRIPTION OF THE DRAWINGS

### Description of the drawings

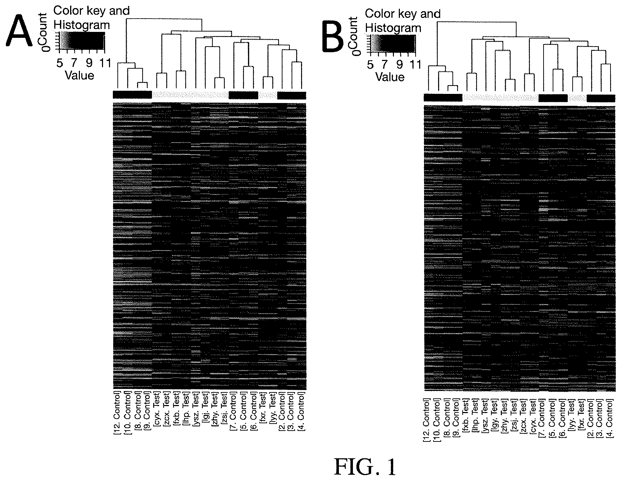

Fig. 1 shows the expression profiles of lncRNAs and mRNAs in aqueous humors of patients with glaucoma;
   A: Cluster analysis diagram of the expression profile of lncRNAs in aqueous humors of patients with glaucoma; B: Cluster analysis diagram of the expression profile of mRNAs in aqueous humors of patients with glaucoma.

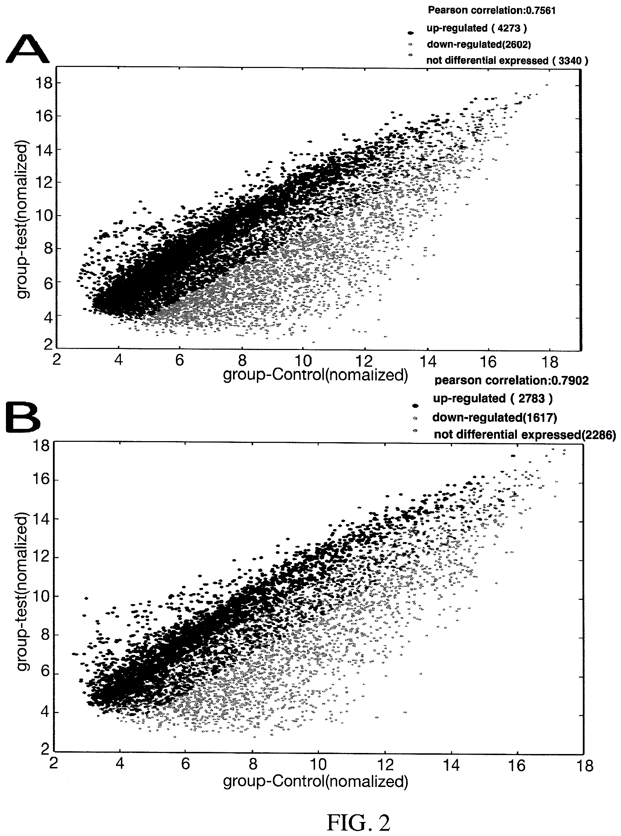

Fig. 2 shows differentially expressed lncRNAs and mRNAs in aqueous humors of patients with glaucoma;
   A: Compared with age-related cataracts, two-fold differentially expressed lncRNAs in aqueous humors of patients with glaucoma; B: Compared with age-related cataracts, two-fold differentially expressed mRNAs in aqueous humors of patients with glaucoma.
Fig. 3 shows CNC analysis diagrams of expressions of lncRNAs and mRNAs in aqueous humors of patients with glaucoma;
   Red dots represent lncRNAs, blue dots represent mRNAs, solid lines represent a positive correlation, and dashed lines represent a negative correlation.
Fig. 4 shows the expression quantities of lncRNAs in aqueous humors of patients with glaucoma and age-related cataracts;
   A-C: scatter plots of expressions of T267384, ENST00000607393, and T342877 in aqueous humors of patients with glaucoma and age-related cataracts; D-F: box plots of expressions of T267384, ENST00000607393, and T342877 in aqueous humors of patients with glaucoma and age-related cataracts.
Fig. 5 shows the expression quantities of lncRNAs in iris tissues of patients with glaucoma and control people;
   A-C: scatter plots of expressions of T267384, ENST00000607393, and T342877 in iris tissues of patients with glaucoma and control people; D-F: box plots of expressions of T267384, ENST00000607393, and T342877 in iris tissues of patients with glaucoma and control people.
Fig. 6 shows the expression quantities of lncRNAs in serums of patients with glaucoma and control people;
   A-C: scatter plots of expressions of T267384, ENST00000607393, and T342877 in serums of patients with glaucoma and control people; D-F: box plots of expressions of T267384, ENST00000607393, and T342877 in serums of patients with glaucoma and control people.
Fig. 7 shows ROC curves of using lncRNAs for the diagnosis of glaucoma in different tissues;
   A: An ROC curve of using T267384 for the diagnosis of glaucoma in aqueous humor, with the area under the curve of 0.998; B: An ROC curve of using ENST00000607393 for the diagnosis of glaucoma in aqueous humor, with the area under the curve of 0.998; C: An ROC curve of using T342877 for the diagnosis of glaucoma in aqueous humor, with the area under the curve of 0.983; D: An ROC curve of using ENST00000607393 for the diagnosis of glaucoma in the iris tissue, with the area under the curve of 0.793; E: An ROC curve of using T267384 for the diagnosis of glaucoma in serum, with the area under the curve of 0.620; F: An ROC curve of using ENST00000607393 for the diagnosis of glaucoma in serum, with the area under the curve of 0.638.

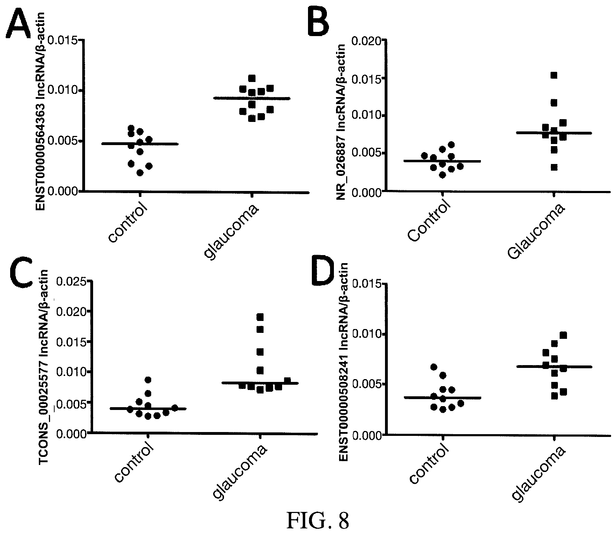

Fig. 8 shows the expression quantities of another group of lncRNAs in aqueous humors of patients with glaucoma and age-related cataracts;
   A-D: scatter plots of expressions of lncRNA: ENST00000564363, NR_026887, TCONS_00025577, and ENST00000508241 in aqueous humors of patients with glaucoma and age-related cataracts.

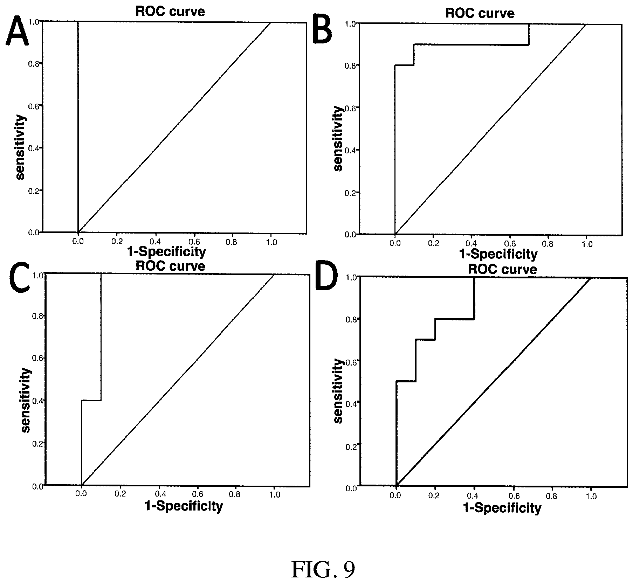

Fig. 9 shows ROC curves of using another group of lncRNAs for the diagnosis of glaucoma in aqueous humor;
   A-D: ROC curves of using lncRNA ENST00000564363, NR_026887, TCONS_00025577, and ENST00000508241 for the diagnosis of glaucoma in aqueous humor samples, with the areas under the ROC curves of 1.000, 0.920, 0.940, and 0.880, respectively.

### DETAILED DESCRIPTION

### Embodiments of the invention

The present invention is further illustrated in conjunction with the following embodiments but is not limited thereto.

### Embodiment 1: Expression profiles of lncRNAs and mRNAs in aqueous humors of patients with glaucoma:

### 1.1 Materials and reagents

### 1.1.1 Main instruments

Common centrifuges and cryogenic centrifuges were purchased from Eppendorf Company; high-speed centrifuges were purchased from Beckman Company; Real-Time PCR instruments were purchased from ABI Company; pipettes and electric pipetting guns were purchased from Eppendorf Company; Q5000 was purchased from Quawell Technology Company; and ice machines were purchased from Sanyan Company.

### 1.1.2 Materials and reagents

Different models of centrifuge tubes and PCR tubes were all purchased from Axygen Company; DEPC water was purchased from Dingguo Changsheng Company; Trizol was purchased from Invitrogen Company; TargetAmp ™1-Round aRNA Amplification kits were purchased from epicentre Company; Transcriptor First Strand cDNA Synthesis kits were purchased from Roche Company; Quick Amp Labeling Kit, One-Color kits were purchased from Agilent Technologies Company; and human lncRNAs microarray chips were purchased from Arraystar Company.

### 1.1.3 Preparation of reagents

Various reagents used for immunoblot were prepared according to the methods provided in Molecular Cloning (Third Editi*on).*

### 1.2 Methods

### 1.2.1 Preparation of aqueous humor samples

The research has been approved by the Ethics Committee of the Second Xiangya Hospital of Central South University. Informed consent was signed with all research subjects before surgery. Before the formal surgical procedure starts, 0.1 ml of an undiluted aqueous humor sample was taken from the limbus corneae with a 1 ml disposable sterile syringe, and immediately injected into an autoclaved 0.5 ml EP tube to be stored in a -80°C low temperature refrigerator in the dark for later use. All enrolled patients were patients with glaucoma and patients with age-related cataracts who needed surgery in our hospital. Inclusion criteria: 1. Primary open-angle glaucoma: having the following four items or having A, D, B or C: A. intraocular tension of more than 21 mmHg; B. glaucomatous optic disc damage and /RNFL defect; C. typical glaucomatous visual field defect; D. opened anterior chamber angle. 2. Normal tension glaucoma: having an optic disc change similar to POAG, RNFL and visual field damage, the intraocular tension being measured within 24h to be all less than or equal to 21 mmHg, and opened anterior chamber angle. 3. Primary angle-closure glaucoma: having a glaucomatous optic disc change, RNFL and visual field damage, the intraocular tension of more than 21 mmHg, and narrowed or closed anterior chamber angle. 4. Age-related cataracts (control group): crystalline lenses having cortical and/or karyotype opacity and/or posterior capsule opacity. Exclusion criteria: 1. accompanied with systemic diseases such as hypertension and diabetes. 2. Secondary glaucoma. 3. Ophthalmic or neurological diseases that may affect vision, optic nerve, or color vision. 4. Reliability criteria for visual field detection: the fixation loss rate, false positive rate and/or false negative rate are more than 25%. 5. Age-related cataracts: complicated, traumatic, and congenital cataracts are excluded and those with other intraocular diseases are excluded, and hypermature cases are excluded.

### 1.2.2 RNA extraction

0.1 ml of aqueous humor was taken and added with 0.5 ml of a Trizol reagent, and 0.1 ml of chloroform was added after shaking vigorously. Then, after centrifugation, precipitation and washing with 75% ethanol, the product was dissolved in 10 µl of DPEC water, and the RNA concentration of each tube was measured by a Q5000 instrument.

### 1.2.3 RNA amplification

RNA was amplified by the TargetAmp ™1-Round aRNA Amplification Kit 103 (epicentre) according to the steps as indicated in the manufacturer's instructions. The general steps are as follows: firstly, a single-stranded cDNA:RNA hybridization product was synthesized from an RNA sample, then the hybridization product was digested into small fragment sequences by an RNase H enzyme, and these small fragment sequences could assist in the synthesis of double-stranded cDNA. Finally, antisense RNA was synthesized by double-stranded cDNA transcription.

### 1.2.4 cDNA synthesis and labeling

cDNA was synthesized by the Transcriptor First Strand cDNA Synthesis Kit (Roche) according to the steps as indicated in the manufacturer's instructions. The synthesized cDNA was labeled by the Quick Amp Labeling Kit, One-Color (Agilent Technologies).

### 1.2.5 Labeling efficiency quality detection

1.5 µl of a labeled cDNA sample was taken, and the fluorescent labeling efficiency was detected by NanoDropND-1000.

### 1.2.6 Chip hybridization

A labeled probe and the high-density chip (Human lncRNA microarray V4.0, Arraystar) were hybridized under standard conditions. A total of 40,173 lncRNAs and 20,730 mRNAs expression levels were detected by the chip.

### 1.2.7 Image collection and data analysis

The fluorescence intensity of the chip was scanned by a GenePix 4000B chip scanner, and the experimental results were converted into digital data to be stored. P value < 0.05 is considered that the difference is statistically significant.

### 1.3 Results

### 1.3.1 Expression profiles of lncRNAs and mRNAs in aqueous humors of patients with glaucoma

An average of 20653 ± 569.9 lncRNAs and 11265 ± 268.3 mRNAs were detected in ten aqueous humor samples of patients with glaucoma. Among them, the number of lncRNAs that were detected in all the ten aqueous humor samples of patients with glaucoma was 11,728, and the number of mRNAs that were detected in all the ten aqueous humor samples of patients with glaucoma was 6686.

### 1.3.2 Differentially expressed lncRNAs and mRNAs in aqueous humors of patients with glaucoma

Compared with age-related cataracts, the number of lncRNAs of which the expression was up-regulated by two folds in the aqueous humors of patients with glaucoma was 4372, and the number of lncRNAs of which the expression was down-regulated by two folds was 2602. Compared with age-related cataracts, the number of mRNAs of which the expression was up-regulated by two folds in the aqueous humors of patients with glaucoma was 2,783, and the number of mRNAs of which the expression was down-regulated by two folds was 1,617.

### 1.4 Results

Because of its ease taking, individual specificity and relatively little impact from other organs of the body, the aqueous humor is a valuable research object for biomarker-related research of eye diseases. Moreover, although many researches on glaucoma-related epidemiological and genetic risk factors have been reported, there are relatively few related researches on aqueous humor as a research object. Therefore, we believe that the aqueous humor has the potential to be applied to the genetics research related to glaucoma in the future. The human aqueous humor sample is small in size due to sampling limitations. To overcome the problem, an RNA amplification step was added before chip hybridization, so as to ensure the smooth progress of the subsequent steps. By using the lncRNAs chip microarray analysis method, 11728 lncRNAs and 6686 mRNAs were detected in ten glaucoma aqueous humor samples, which were significantly different from the expression profiles of lncRNAs and mRNAs in the aqueous humors of patients with age-related cataracts. Therefore, the expression profiles of lncRNAs and mRNAs in the aqueous humors exhibit individual specificity and disease specificity. As far as we know, this is the first research on the expression profiles of lncRNAs and mRNAs related to glaucoma aqueous humors.

### Embodiment 2: Confirming of correlation between specific lncRNAs and mRNAs in aqueous humor by CNC analysis

To further investigate the possible functions of lncRNAs expressed in aqueous humors of patients with glaucoma, lncRNAs with a significant correlation with mRNAs expression of glaucoma-related genes were confirmed by CNC (coding-noncoding gene co-expression) analysis. CNS analysis is an analytical method that links lncRNA to mRNA by co-expression data of lncRNA and mRNA. Through CNC analysis, mRNA with the same expression pattern as a certain lncRNA can be found. Through the functions of these mRNAs, lncRNA can be linked to specific signaling pathways or disease states, thereby conveniently predicting the functions of lncRNA and revealing its mechanism of action.

### 2.1 Methods

The mRNAs that were differentially expressed in the aqueous humor and had a correlation with the occurrence and development of glaucoma were selected, and the expression data of these mRNAs in the aqueous humors of different patients with glaucoma were averaged; the Pearson correlation coefficient (PCC) and the false discovery rate (FDR) between the data after standardization of selected mRNAs and the differentially expressed lncRNAs related data in the aqueous humors of patients with glaucoma were solved; records with PCC ≥ 0.90 and FDR ≤ 0.05 were selected; and drawing was carried out by a Cytoscape 2.8.3 tool using related records.

### 2.2 Results

Compared with age-related cataracts, there were the following ten mRNAs that are differentially expressed in the aqueous humors of patients with glaucoma and have the correlation with the occurrence and development of glaucoma: bone morphogenetic protein 2 (BMP2), ependymin related gene 1 (EPDR1), transforming growth factor beta 1 (TGFB1), forkhead box protein E3 (FOXE3), growth hormone secretagogue receptor (GHSR), forkhead box protein Cl (FOXCl), transmembrane and coiled-coil domains 1 (TMCO1), pleckstrin homology domain containing A7 (PLEKHA7), optineurin (OPTN) and integrin subunit beta 5 (ITGB5). In the aqueous humors of patients with glaucoma, there were ten lncRNAs with the Pearson correlation coefficient greater than 0.9 and the false discovery rate of less than 0.05 related to the expression of these mRNAs.

### Embodiment 3

In the ten lncRNAs in Embodiment 2 verified in the aqueous humors of patients with glaucoma and age-related cataracts by qRT-PCR, the differential expression quantities of seven lncRNAs in the two types of aqueous humors have statistical difference, and the differential expression quantities of three lncRNAs in the two types of aqueous humors have no statistical difference. Then T267384, ENST00000607393 and T342877 with the most significant differential expression quantities were selected for further verification.

### Embodiment 4

A qRT-PCR detection method was used to verify the expression levels of three lncRNA molecules with the most significant difference in Embodiment 3 in aqueous humors, iris tissues and serums, and analyze the difference of expression quantities between patients with glaucoma and the normal people.

### 4.1 Materials and reagents

### 4.1.1 Main instruments

Common centrifuges and cryogenic centrifuges were purchased from Eppendorf Company; high-speed centrifuges were purchased from Beckman Company; Real-Time PCR instruments were purchased from ABI Company; pipettes and electric pipetting guns were purchased from Eppendorf Company; Q5000 was purchased from Quawell Technology Company; and ice machines were purchased from Sanyan Company.

### 4.1.2 Materials and reagents

Different models of centrifuge tubes and PCR tubes were all purchased from Axygen Company; DEPC water was purchased from Dingguo Changsheng Company; Trizol was purchased from Invitrogen Company; and reverse transcription kits and fluorescent quantitative kits were all purchased from Roche Company.

### 4.1.3 Preparation of reagents

Various reagents used for immunoblot were prepared according to the methods provided in Molecular Cloning (Third Editi*on).*

### 4.2 Methods

### 4.2.1 Preparation of aqueous humor, serum and iris samples

The aqueous humor samples were prepared as described in the step 1.2.1. The serum samples were taken from patients with glaucoma and people from the healthy control group. The inclusion and exclusion criteria for patients with glaucoma were the same as those described in 1.2.1. The serum from the control group was taken from healthy people matched with patients in the glaucoma group in age and gender. The iris sample was taken from the glaucoma patient group and the control group. The inclusion and exclusion criteria for patients with glaucoma were the same as those described in 1.2.1. The iris sample from the control group was taken from the iris tissues of cornea donors of the Xiangya Second Hospital, which were matched with the patients with glaucoma in age and gender.

### 4.2.2 RNA extraction

0.1 ml of aqueous humor or iris tissue was added with 0.5 ml of a Trizol reagent, and 0.1 ml of chloroform was added after shaking vigorously. Then, after centrifugation, precipitation and washing with 75% ethanol, the product was dissolved in 20 µl of DPEC water, and the RNA concentration of each well was measured by a Q5000 instrument. Peripheral venous blood was allowed to stand for 30 minutes, and placed in a centrifuge to be centrifuged at 3200 rpm for 10 minutes, a supernatant serum was taken by a sterile pipette, and RNA was extracted by the same step as above.

### 4.2.3 qRT-PCR

According to the manufacturer's instructions, RNA was reversely transcribed into cDNA by a reverse transcription enzyme firstly, and then the cDNA was amplified by a PCR method, and the quantity of the quantitatively amplified product was detected in real time by measuring a fluorescence intensity signal.

### 4.2.2 Data Analysis

Data was processed by SPSS19.0 version statistical software. Measurement data was expressed as median ± quartile spacing, and glaucoma/cataract represented the median ratio of the two groups and Mann-whitney U test was performed by comparison. P value < 0.05 was considered that the difference was statistically significant.

### 4.3 Results

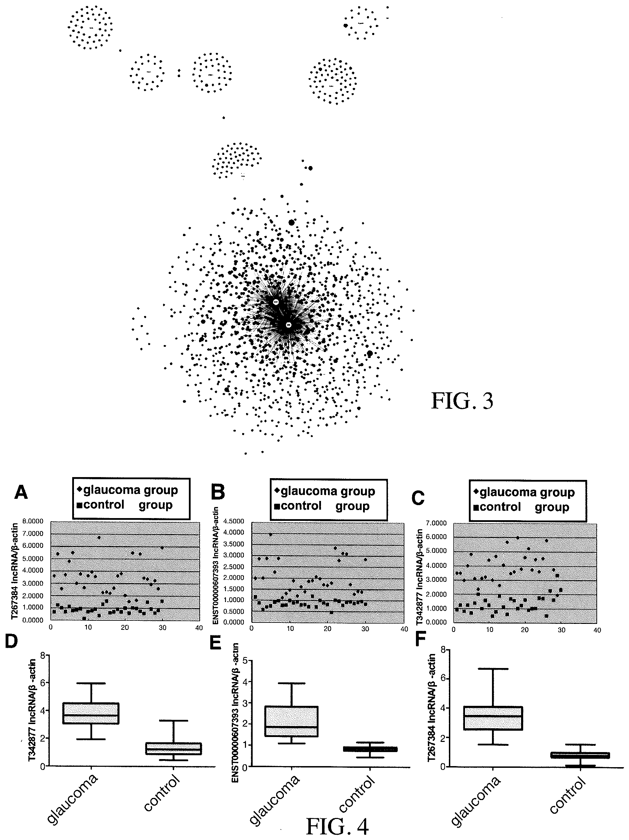

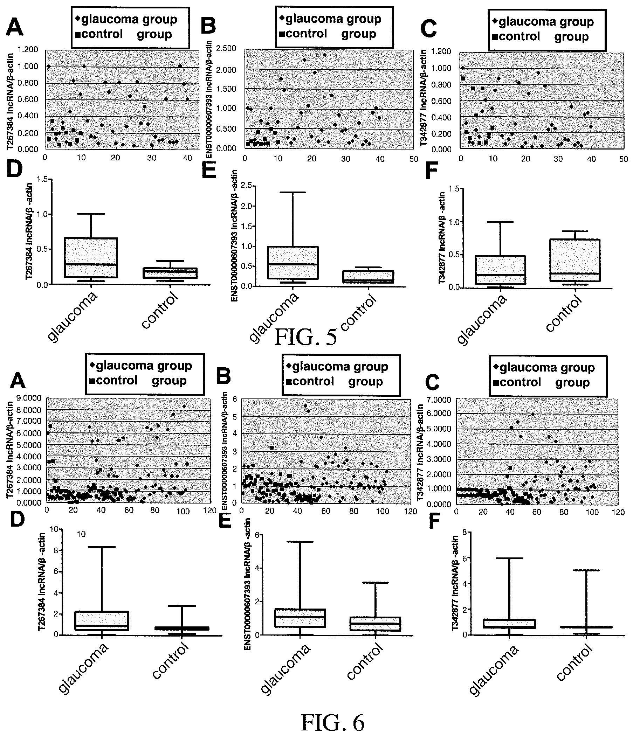

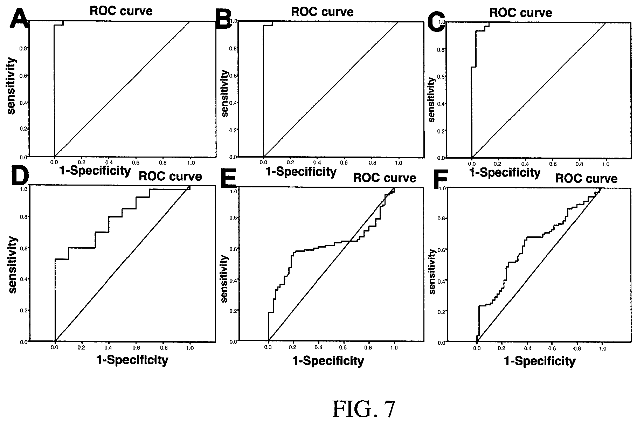

To further expand the aqueous humor sample size to verify the expression levels of the related lncRNAs and to explore the expression quantities of specific lncRNAs in the serums and irises of patients with glaucoma, qRT-PCR was used to detect the expression levels of T267384, ENST00000607393 and T342877 in 60 aqueous humor samples (30 aqueous humor samples of patients with glaucoma, and 30 aqueous humor samples of age- and gender-matched patients with age-related cataracts), 50 iris tissues (40 iris tissue samples of patients with glaucoma, and 10 iris tissues of age- and sex-matched cornea donors) and 158 serum samples (103 serum samples of patients with glaucoma, and 55 serum samples of age- and sex-matched healthy people). The results show that the expression quantities of three lncRNAs have statistical difference in the two types of aqueous humor samples, and the expression quantities thereof in the aqueous humors of patients with glaucoma are 4.4036, 2.1467 and 2.9692 times larger than those in the aqueous humors of patients with age-related cataracts; the expression quantities of ENST00000607393 in the two types of iris tissues have statistical difference, the expression quantity thereof in the iris tissues of patients with glaucoma is 3.3436 times larger than that in the iris tissues from the control group, while the expression quantities of T267384 and T342877 have no statistical difference; the expression quantities of T267384 and ENST00000607393 have statistical difference in the two serum samples, and the expression quantities thereof in the serums of patients with glaucoma were 1.2878 and 1.5301 times larger than those in the serums from the control group, while the expression quantity of T342877 has no statistical difference. (Tables 1, 2, and 3) (Figs. 4, 5 and 6). To further understand the effectiveness of these three lncRNAs in the diagnosis of glaucoma in different tissues, the receiver operating characteristic (ROC) curves (Fig. 7) were drawn by using the obtained data. It is found that the areas under the ROC curves of T267384, ENST00000607393 and T342877 for the diagnosis of glaucoma in the aqueous humor samples are 0.998, 0.998 and 0.983, respectively; the area under the ROC curve of ENST00000607393 for the diagnosis of glaucoma in iris tissue is 0.793; the areas under the ROC curves of T267384 and ENST00000607393 for the diagnosis of glaucoma in serum samples are 0.620 and 0.638, respectively. When the diagnostic values of T267384, ENST00000607393, and T342877 in the aqueous humors are set to 1.5437, 1.1485, and 2.1052, the sensitivity/specificity of diagnosis is 0.967/0.967, 0.967/0.967, and 0.967/0.900, sequentially; when the diagnostic value of ENST00000607393 in the iris tissues is set to 0.2470, the sensitivity/specificity of diagnosis is 0.700/0.700; when the diagnostic values of T267384 and ENST00000607393 in serums are set to 0.7191 and 0.8376, the sensitivity/specificity of diagnosis is 0.612/0.600 and 0.680/0.600.

**Table 1 Expression Quantities of Three lncRNAs in Aqueous Humors**

| lncRNAs | Glaucoma group | Cataract group | Glaucoma/cataract | P value |
|---|---|---|---|---|
| T267384 | 3.4793±1.2848 | 0.7901±0.3129 | 4.4036 | 0.000 |
| ENST00000607393 | 1.8700±1.3398 | 0.8711±0.1666 | 2.1467 | 0.000 |
| T342877 | 3.6545±1.4040 | 1.2308±0.7356 | 2.9692 | 0.000 |

**Table 2 Expression Quantities of Three lncRNAs in Iris Tissues**

| lncRNAs | Glaucoma group | Control group | Glaucoma/cataract | P value |
|---|---|---|---|---|
| T267384 | 0.2835±0.5452 | 0.1875±0.1097 | 1.5120 | 0.121 |
| ENST00000607393 | 0.5537±0.7749 | 0.1656±0.2401 | 3.3436 | 0.005 |
| T342877 | 0.2028±0.3966 | 0.2269±0.5187 | 0.8938 | 0.482 |

**Table 3 Expression Quantities of Three lncRNAs in Serums**

| lncRNAs | Glaucoma group | Control group | Glaucoma/cataract | P value |
|---|---|---|---|---|
| T267384 | 0.8944±1.6348 | 0.6945±0.1908 | 1.2878 | 0.013 |
| ENST00000607393 | 1.0905±1.0336 | 0.7127±0.7648 | 1.5301 | 0.004 |
| T342877 | 0.6520±0.6056 | 0.6503±0.0527 | 1.0026 | 0.280 |

**Table 4 Sensitivity and Specificity of Diagnosis of Three lncRNAs in Different Tissues**

| Cut-off values | Sensitivity | Specificity |
|---|---|---|
| 1.5437 (T267384 aqueous humor) | 0.967 | 0.967 |
| 1.1485 (ENST00000607393 aqueous humor) | 0.967 | 0.967 |
| 2.1052 (T342877 aqueous humor) | 0.967 | 0.900 |
| 0.2470 (ENST00000607393 iris) | 0.700 | 0.700 |
| 0.7191 (T267384 serum) | 0.612 | 0.600 |
| 0.8376 (EHST00000607393 serum) | 0.680 | 0.600 |

### Embodiment 5

In the ten lncRNAs in Embodiment 2 verified in the aqueous humors of patients with glaucoma and age-related cataracts by qRT-PCR, the differential expression quantities of seven lncRNAs in the two types of aqueous humors have statistical difference, and the differential expression quantities of three lncRNAs in the two types of aqueous humors have no statistical difference. Then another group of lncRNAs: ENST00000564363, NR _026887, TCONS_00025577 and ENST00000508241 having statistical difference were selected for further verification.

### Embodiment 6

A qRT-PCR detection method was used to verify the expression levels of lncRNA: ENST00000564363, NR_026887, TCONS_00025577 and ENST00000508241 molecules in aqueous humors, and analyze the difference of expression quantities between patients with glaucoma and people in the control group.

### 6.1 Materials and reagents

### 6.1.1 Main instruments

Common centrifuges and cryogenic centrifuges were purchased from Eppendorf Company; high-speed centrifuges were purchased from Beckman Company; Real-Time PCR instruments were purchased from ABI Company; pipettes and electric pipetting guns were purchased from Eppendorf Company; Q5000 was purchased from Quawell Technology Company; and ice machines were purchased from Sanyan Company.

### 6.1.2 Materials and reagents

Different models of centrifuge tubes and PCR tubes were all purchased from Axygen Company; DEPC water was purchased from Dingguo Changsheng Company; Trizol was purchased from Invitrogen Company; and reverse transcription kits and fluorescent quantitative kits were all purchased from Roche Company.

### 6.1.3 Preparation of reagents

Various reagents used for immunoblot were prepared according to the methods provided in Molecular Cloning (Third Editi*on).*

### 6.2 Methods

### 6.2.1 Preparation of aqueous humor samples

The aqueous humor samples were prepared as described in THE step 1.2.1.

### 6.2.2 RNA extraction

0.1 ml of aqueous humor was added with 0.5 ml of a Trizol reagent, and 0.1 ml of chloroform was added after shaking vigorously. Then, after centrifugation, precipitation and washing with 75% ethanol, the product was dissolved in 20 µl of DPEC water, and the RNA concentration of each well was measured by a Q5000 instrument.

### 6.2.3 qRT-PCR

According to the manufacturer's instructions, RNA was reversely transcribed into cDNA by a reverse transcription enzyme firstly, then the cDNA was amplified by a PCR method, and the quantity of the quantitatively amplified product was detected in real time by measuring a fluorescence intensity signal.

### 6.2.4 Data Analysis

Data was processed by SPSS19.0 version statistical software. Measurement data was expressed as median ± quartile spacing, and glaucoma/cataract represented the median ratio of the two groups and Mann-whitney U test was performed by comparison. P value < 0.05 was considered that the difference was statistically significant.

### 6.3 Results

To further expand the aqueous humor sample size to verify the expression levels of the related lncRNAs and to explore the expression quantities of specific lncRNAs in the aqueous humors of patients with glaucoma, qRT-PCR was used to detect the expression levels of ENST00000564363, NR_026887, TCONS_00025577, and ENST00000508241 in 20 aqueous humor samples (10 aqueous humor samples of patients with glaucoma, and 10 aqueous humor samples of age- and gender-matched patients with age-related cataracts). The results show that the expression quantities of ENST00000564363, NR 026887, TCONS 00025577, and ENST00000508241 have statistical difference in the two types of aqueous humor samples, and the expression quantities thereof in the aqueous humors of patients with glaucoma are 1.6200, 1.9500, 1.9302, and 1.8378 times larger than those in the aqueous humors of patients with age-related cataracts (Table 5) (Fig. 8). To further understand the effectiveness of ENST00000564363, NR_026887, TCONS_00025577, and ENST00000508241 for the diagnosis of glaucoma in aqueous humors, the receiver operating characteristic (ROC) curves (Fig. 9) were drawn by using the obtained data. It is found that the areas under the ROC curves of ENST00000564363, NR 026887, TCONS 00025577, and ENST00000508241 for the diagnosis of glaucoma in the aqueous humor samples are 1.000, 0.920, 0.940 and 0.880, respectively. When the diagnostic values of ENST00000564363, NR 026887, TCONS 00025577, and ENST00000508241 in the aqueous humor are set to 0.0068, 0.0056, 0.0074 and 0.0048, the sensitivity/specificity of diagnosis is 1.000/1.000, 0.900/0.900, 0.900/0.900 and 0.800/0.800, respectively (Table 6).

**Table 5 Expression Quantities of lncRNAs in Aqueous Humors**

| lncRNAs | Glaucoma group | Cataract group | Glaucoma/cataract | P value |
|---|---|---|---|---|
| ENST00000564363 | 0.0081±0.0020 | 0.0050±0.0013 | 1.6200 | 0.001 |
| NR_ 026887 | 0.0078±0.0021 | 0.0040±0.0015 | 1.9500 | 0.001 |
| TCONS 00025577 | 0.0083±0.0050 | 0.0043±0.0018 | 1.9302 | 0.001 |
| ENST00000508241 | 0.0068±0.0027 | 0.0037±0.0017 | 1.8378 | 0.004 |

**Table 6 Sensitivity and Specificity of Diagnosis of lncRNAs in Aqueous Humors**

| Cut-off values | Sensitivity | Specificity |
|---|---|---|
| 0.0068 (ENST00000564363) | 1.000 | 1.000 |
| 0.0056 (NR 026887) | 0.900 | 0.900 |
| 0.0074 (TCONS 00025577) | 0.900 | 0.900 |
| 0.0048 (ENST00000608241) | 0.800 | 0.800 |

## Claims

1. A molecular marker lncRNAs ENST00000607393 for glaucoma diagnosis, having a sequence shown in SEQ ID NO: 1.

2. Application of a product for detecting the expression level of lncRNAs ENST00000607393 in preparation of a tool for glaucoma diagnosis.

3. The application according to claim 2, wherein the product for detecting the expression level of lncRNAs ENST00000607393 comprises a preparation for detecting the expression level of lncRNAs ENST00000607393 by real-time fluorescent quantitative PCR.

4. The application according to claim 3, wherein the preparation for detecting the expression level of lncRNAs ENST00000607393 by real-time fluorescent quantitative PCR comprises primers specifically amplifying lncRNAs ENST00000607393.

5. The application according to claim 4, wherein the sequences of the primers specifically amplifying lncRNAs ENST00000607393 for detecting the expression level of lncRNAs ENST00000607393 by real-time fluorescent quantitative PCR are as follows:
F:5'-GGCGTCTGTTTGTTTATTGGC-3',
R:5'-TCACTTTTGCTTGGGGAGATT-3'.

6. A kit for glaucoma diagnosis, wherein the kit comprises a reagent for detecting the expression level of IncRNAs ENST00000607393.

7. The kit for glaucoma diagnosis according to claim 6, wherein the kit comprises a reagent for detecting the expression level of lncRNAs ENST00000607393 by real-time fluorescent quantitative PCR.

8. The kit for glaucoma diagnosis according to claim 7, wherein the reagent for detecting the expression level of lncRNAs ENST00000607393 by real-time fluorescent quantitative PCR comprises primers specifically amplifying lncRNAs ENST00000607393 by real-time fluorescent quantitative PCR.

9. The kit for glaucoma diagnosis according to claim 7 or 8, wherein the reagent for detecting the expression level of lncRNAs ENST00000607393 by real-time fluorescent quantitative PCR comprises a pair of primers specifically amplifying lncRNAs ENST00000607393 by real-time fluorescent quantitative PCR, of which the primer sequences are as follows:
F:5'-GGCGTCTGTTTGTTTATTGGC-3',
R:5'-TCACTTTTGCTTGGGGAGATT-3'.

10. A molecular marker lncRNAs ENST00000508241 for glaucoma diagnosis, having a sequence shown in SEQ ID NO: 4.

11. Application of a product for detecting the expression level of lncRNAs ENST00000508241 in preparation of a tool for glaucoma diagnosis.

12. The application according to claim 11, wherein the product for detecting the expression level of lncRNAs ENST00000508241 comprises a preparation for detecting the expression level of lncRNAs ENST00000508241 by real-time fluorescent quantitative PCR.

13. The application according to claim 12, wherein the preparation for detecting the expression level of lncRNAs ENST00000508241 by real-time fluorescent quantitative PCR comprises primers specifically amplifying lncRNAs ENST00000508241.

14. The application according to claim 13, wherein the sequences of the primers specifically amplifying lncRNAs ENST00000508241 for detecting the expression level of lncRNAs ENST00000508241 by real-time fluorescent quantitative PCR are as follows:
F:5'TTTGGGTTTGGCTGTTACTCA3'
R:5'GACCACATTTCCCAAGAACACT3'.

15. A kit for glaucoma diagnosis, wherein the kit comprises a reagent for detecting the expression level of lncRNAs ENST00000508241.

16. The kit for glaucoma diagnosis according to claim 15, wherein the kit comprises a reagent for detecting the expression level of lncRNAs ENST00000508241 by real-time fluorescent quantitative PCR.

17. The kit for glaucoma diagnosis according to claim 16, wherein the reagent for detecting the expression level of lncRNAs ENST00000508241 by real-time fluorescent quantitative PCR comprises primers specifically amplifying lncRNAs ENST00000508241 by real-time fluorescent quantitative PCR.

18. The kit for glaucoma diagnosis according to claim 16 or 17, wherein the reagent for detecting the expression level of lncRNAs ENST00000508241 by real-time fluorescent quantitative PCR comprises a pair of primers specifically amplifying lncRNAs ENST00000508241 by real-time fluorescent quantitative PCR, of which the primer sequences are as follows:
F:5'TTTGGGTTTGGCTGTTACTCA3'
R:5'GACCACATTTCCCAAGAACACT3'.
